# EUROPEAN PATENT APPLICATION

(11) **EP 1 584 287 A1**
(43) Date of publication of application: **12.10.2005**
(21) Application number: 05007588.6
(22) Date of filing: 07.04.2005
(51) Int. Cl.: A61B 5/00, A61B 5/02

(54) **Clip-type sensor having integrated biasing and cushioning means**

(30) Priority: 07.04.2004 US 821259
(71) Applicant: Elekon Industries USA, Inc., Torrance, California 90503 (US)
(72) Inventor: Dietiker, Thomas, Rancho Palos Verdes, California 90503 (US)
(74) Representative: Schwanhäusser, Gernot

(57) **Abstract**

A sensor device for non-invasively measuring a physiological parameter of a patient, such as a pulse oximeter for measuring blood oxygen levels of a patient In a preferred embodiment, the device comprises a first device portion and a second device portion pivotally connected to the first device portion to define a clamping end of the device. A sensing mechanism is in communication with the clamping end of the device for sensing at least one parameter utilized to determine the blood oxygen level of a patient. A resilient member is disposed between the device portions for biasing the device portions toward each other at the clamping end of the device for clamping an appendage of the patient therebetween. The resilient member further provides a cushion for the appendage at the clamping end of the device. The resilient member thus provides both a biasing component and a cushioning component in a one-piece configuration, thereby eliminating the need for a separate biasing and cushioning elements.

## Description

### BACKGROUND OF THE INVENTION

### 1. Field of the Invention

The present invention generally relates to clip-type sensor devices for use in measuring a physiological parameter of a patient. More particularly, this invention relates to a clip-type sensor device, such as a reusable pulse oximetry (SPO₂) finger sensor, having integrated biasing and cushioning means.

### 2. Background

A common non-invasive medical technique used to monitor blood oxygen levels is pulse oximetry. This technique takes advantage of the fact that light transmissivity and color of blood is a function of the oxygen saturation of heme in the blood's hemoglobin. Heme that is saturated with oxygen appears bright red because heme is relatively permeable to red light when it is saturated. Heme that is not saturated, or deoxygenated, appears dark and bluish as it is less permeable to red light Based on these concepts, a pulse oximeter system measures the oxygen content of arterial blood by first illuminating the blood with red and infrared radiation and determining the corresponding amounts of red and infrared radiation that are absorbed by the heme in the blood. By applying these measurements to other known information, blood oxygen levels can be determined.

A pulse oximeter typically includes an optical sensor that detects light which is passed through an appendage of a patient, typically a patient's finger, ear lobe, nasal septum or other portion of the body through which light can be easily transmitted. The amounts of light detected at various wave lengths are then used to determine arterial oxygen saturation. The optical sensor is typically in the form of a light emitter and a corresponding light detector. The pulse oximeter generally employs a means for holding the emitter and detector combination relative to the patient's body, One common means is a clip, which is employed in a clip-type sensor. The clip- type sensor typically includes two hingedly connected housings onto which the emitter and detector are mounted. The clip-type sensor is releasably attached to a patient's appendage so that the appendage is isolated between the two housings. The emitter, typically a diode, is mounted to one of the housings and emits light at a certain wave length through the appendage. The detector is mounted opposite the emitter to the other housing and detects the amount of light that is transmitted through the appendage at various wavelengths.

Although the general concept of a clip-type sensor device is known, there is a need for improvement in design and construction, especially from the standpoints of manufacturability, robustness of design, and overall costs. The present invention provides an improved clip-type sensor device that incorporates a resilient member that unitarily biases the clamping device and provides a cushion for a patient's appendage. Such a device provides the advantages of a more robust design, ease of manufacturability and reduction in costs associated with manufacture of the device. Other advantages will also be apparent from the written specification, drawings and claims herein.

### SUMMARY OF THE INVENTION

The present invention generally provides a sensor device for non-invasively measuring a physiological parameter of a patient, such as a pulse oximeter for measuring blood oxygen levels of a patient. In a preferred embodiment, the device comprises a first device portion and a second device portion pivotally connected to the first device portion to define a clamping end of the device. A sensing mechanism is in communication with the clamping end of the device for sensing at least one parameter utilized to determine the blood oxygen level of a patient. A resilient member is disposed between the device portions for biasing the device portions toward each other at the clamping end of the device for clamping an appendage of the patient therebetween. The resilient member further provides a cushion for the appendage at the clamping end of the device.

According to a particular aspect of the invention, the resilient member comprises a bias portion distally disposed from the clamping end of the device and a cushion portion proximally disposed to the clamping end of the device.

According to another aspect of the invention, the resilient member comprises a first cushion portion in communication with the first device portion at the clamping end of the device; a second cushion portion in communication with the second device portion at the clamping end of the device; and a bias portion in communication with both device portions proximate the pivotal connection therebetween.

According to yet another aspect, the resilient member comprises an elastomeric material. The resilient member can be made from a material selected from the group consisting essentially of liquid silicon robber, thermoplastic elastomers, polyolefin elastomers, thermoplastic rubbers, natural rubbers, and methanes.

According to yet another aspect, a resilient member for use with a clip-type sensor is provided. The member comprises a bias portion that fits between a first portion and a second portion of the clip-type sensor to bias the portions into a clamped position; and a cushion portion integrally formed with the bias portion that fits between the first portion and the second portion of the clip-type sensor to cushion a finger clamped by the sensor.

In accordance with the principles of the present invention, the resilient member provides both a biasing means and a cushioning means in a one-piece configuration, thereby eliminating the need for a separate biasing and cushioning means.

Other features and aspects of the invention will be apparent from the written specification, drawings and claims herein.

### BRIEF DESCRIPTION OF THE DRAWINGS

**FIG. 1** is a first perspective view of an embodiment of a sensor device in accordance with the principles of the present invention;
**FIG. 2** is a second perspective view of the embodiment of FIG. 1;
**FIG. 3** is a side elevational view of the embodiment of FIG. 1; and
**FIG. 4** is an end elevational view of the embodiment of FIG. 1.

### DETAILED DESCRIPTION OF THE PREFERRED EMBODIMENTS

While this invention is susceptible of embodiment in many different forms, there is shown in the drawings and will herein be described in detail one or more embodiments with the understanding that the present disclosure is to be considered as an exemplification of the principles of the invention and is not intended to limit the invention to the embodiments illustrated.

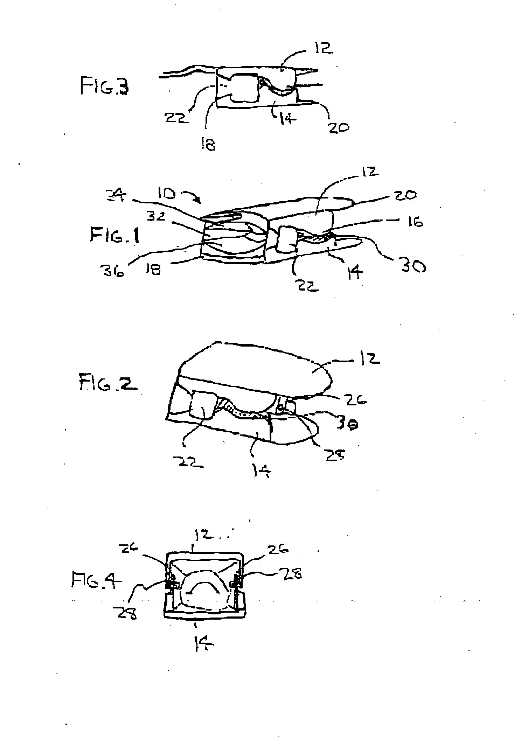

Referring to FIGS. 1-4, an embodiment in accordance with the principles of the present invention is shown in the form of a reusable SP02 finger sensor device 10. It is to be understood, however, that the principles of the present invention may be applied to any type of clip-type sensor device for use with an appendage of a patient. Referring again to FIGS. 1-4, the device 10 comprises a first, or top, portion 12 and a second, or bottom, portion 14 pivotally connected together at a hinge16 to define a clamping end 18 and an actuation end 20 of the device 10. A resilient member 22 is interposed between the portions 12 and 14 and provides a biasing means therebetween, which exerts a biasing force against the device portions 12 and 14 and, in cooperation with the hinge 16, draws the device portions 12 and 14 together at the clamping end 18 in a normally closed position.

The sensor device 10 is shown in FIGS. 1-4 in a closed position. To receive a patient's finger, the device portions 12 and 14 must be drawn together at the actuation end 20 of the device 10, against the bias provided by the resilient member 22, to cause the device portions 12 and 14 at the clamping end 18 to separate and allow the patient's finger to be inserted therebetween. Referring to FIGS. 2 and 4, the device 10 may include at least one track 26 and at least one pin 28 disposed within the track 26 to guide movement of the device portions 12 and 14 in relation to each other. The track 26 in combination with the pin 28 can provide limits to the extent of opening and closing of the device portions 12 and 14.

Referring again to FIGS. 1-4, it can be seen that the resilient member 22 is a one-piece member that extends between the top and bottom portions 12 and 14. In a preferred embodiment, the resilient member 22 comprises a bias portion 30 distally disposed from the clamping end 18 of the device 10 and a cushion portion 32 proximally disposed to the clamping end 18 of the device 10. As shown in FIG. 1, the cushion portion 32 further comprises a first cushion portion 34 and a second cushion portion 36 divergently opposed to each other to allow a patient's finger to be inserted therebetween. The cushion portions 34 and 36 of the resilient member 22 provide cushion to the patient's finger when it is clamped thereto. The cushion portions 34 and 36 also provide a tactile surface that grips a patient's finger to facilitate a secure fit. The first cushion portion 34 is in communication with the first device portion 12 at the clamping end of the device 10. The second cushion portion 36 is in communication with the second device portion 14 at the clamping end 18 of the device 10. In an embodiment, the cushion portions 34 and 36 can be respectively secured to the device portions 12 and 14 by any number of means, such as an adhesive, for example.

As shown in FIGS. 1-3, the bias portion 30 of the resilient member 22 is in communication with both device portions 12 and 14 proximate the pivotal connection therebetween to provide the biasing means between the device portions 12 and 14. The integral formation of the biasing portion 30 and the cushion portion 32 embodied in the resilient member 22 eliminates the need to provide a biasing member, such as a spring or clip, separate from a cushioning member. Thus, this one-piece biasing/cushioning configuration simplifies the design, manufacturability and assembly of the device 10.

The resilient member 22 may be made from materials such as liquid silicon rubber, thermoplastic elastomers, polyolefin elastomers, thermoplastic rubbers, natural rubbers, and urethanes, or any other material known to those skilled in the art that is suitable for providing cushioning properties and that can act as a biasing means. The resilient member 22 is preferably made from an elastomeric material that provides spring-like elastic properties while also providing a relatively soft tactile feel when forced into contact with a patient's finger via the device portions 12 and 14. The elastomeric material also provides a tactile surface to grip the patient's finger and secure the device 10 into place during its use.

The integrated biasing and cushioning aspects of the resilient member 22 facilitates a more robust design, ease of manufacturability, and reduction in costs associated with manufacture of the device 10.

While specific embodiments have been illustrated and described, numerous modifications may come to mind without significantly departing from the spirit of the invention, and the scope of protection is only limited by the scope of the accompanying Claims.

## Claims

**1.** A sensor device for non-invasively measuring a blood oxygen level of a patient, the device comprising:
a first device portion;
a second device portion pivotally connected to the first device portion to define a clamping end of the device;
a sensing mechanism in communication with the clamping end of the device for sensing at least one parameter utilized to determine the blood oxygen level of a patient; and
a resilient member disposed between the device portions for biasing the device portions toward each other at the clamping end of the device for clamping an appendage of the patient therebetween, the resilient member further providing a cushion for the appendage at the clamping end of the device.

**2.** The device according to claim 1, wherein the resilient member further comprises:
a bias portion distally disposed from the clamping end of the device; and
a cushion portion proximally disposed to the clamping end of the device.

**3.** The device according to claim 1, wherein the resilient member comprises:
a first cushion portion in communication with the first device portion at the clamping end of the device;
a second cushion portion in communication with the second device portion at the clamping end of the device; and
a bias portion in communication with both device portions proximate the pivotal connection therebetween.

**4.** The device according to claim 1, wherein the resilient member comprises an elastomeric material.5. The device according to claim 1, wherein the resilient member is made from a material selected from the group consisting essentially of liquid silicon rubber, thermoplastic clastomers, polyolefin elastomers, thermoplastic rubbers, natural rubbers, and urethanes.

**6.** The device according to claim 1, wherein one of the device portions includes at least one track and the other of the device portions includes at least one pin disposed within the track to guide movement of the device portions in relation to each other.

**7.** A sensor device for non-invasively measuring a blood oxygen level of a patient, the device comprising:
a first device portion;
a second device portion pivotally connected to the first device portion to define a clamping end of the device and an actuation end of the device;
a sensing mechanism in communication with the clamping end of the device for sensing at least one parameter utilized to determine the blood oxygen level of a patient; and
a resilient member having a cushion portion disposed between the device portions proximate the clamping end of the device and a bias portion disposed between the device to bias the device portions toward each other at the clamping end of the device.

**8.** The device of claim 7, wherein the cushion portion comprises a first cushion portion in communication with the first device portion and a second cushion portion in communication with the second device portion.

**9.** The device according to claim 7, wherein the resilient member comprises an elastomeric material.

**10.** The device according to claim 7, wherein the resilient member is made from a material selected from the group consisting essentially of liquid silicon rubber, thermoplastic elastomers, polyolefin elastomers, thermoplastic rubbers, natural rubbers, and urethanes.

**11.** The device according to claim 7, wherein one of the device portions includes at least one track and the other of the device portions includes at least one pin disposed within the track to guide movement of the device portions in relation to each other.

**12.** A resilient member for use with a clip-type sensor, the member comprising:
a bias portion that fits between a first portion and a second portion of the clip-type sensor to bias the portions into a clamped position; and
a cushion portion integrally formed with the bias portion that fits between the first portion and the second portion of the clip-type sensor to cushion a finger clamped by the sensor.

**13.** The resilient member according to claim 12, wherein the cushion portion further comprises a first cushion portion and a second cushion portion divergently opposed to each other.

**14.** The resilient member according to claim 12, wherein the resilient member comprises an elastomeric material.

**15.** The resilient member according to claim 12, wherein the resilient material is selected from the group consisting essentially of liquid silicon rubber, thermoplastic elastomers, polyolefin elastomers, thermoplastic rubbers, natural rubbers, and urethanes.
